Europäisches Patentamt

European Patent Office

Office européen des brevets

(19)

(11) Veröffentlichungsnummer: **0 098 957**

**A2**

(12) **EUROPÄISCHE PATENTANMELDUNG**

(21) Anmeldenummer: **83105418.4**

(22) Anmeldetag: **01.06.83**

(51) Int. Cl.³: **C 07 D 277/68**

(30) Priorität: **11.06.82 DE 3221938**

(43) Veröffentlichungstag der Anmeldung:
**25.01.84 Patentblatt 84/4**

(84) Benannte Vertragsstaaten:
**CH DE FR GB LI**

(71) Anmelder: **CASSELLA Aktiengesellschaft
Hanauer Landstrasse 526
D-6000 Frankfurt am Main 61(DE)**

(72) Erfinder: **Kussmaul, Ulrich, Dr.
Tannenweg 39a
D-6367 Karben 1(DE)**

(72) Erfinder: **Müller, Rolf, Dr.
Dornbachweg 3
D-6367 Karben(DE)**

(74) Vertreter: **Urbach, Hans-Georg, Dr. et al,
Hanauer Landstrasse 526
D-6000 Frankfurt am Main 61(DE)**

(54) Verfahren zur Herstellung von kristallinem 2,6-Dichlor-benzothiazol.

(57) Bei dem Verfahren zur Herstellung von kristallisiertem 2,6-Dichlorbenzothiazol wird geschmolzenes 2,6-Dichlorbenzothiazol mit Wasser vereinigt und danach auskristallisiertes 2,6-Dichlorbenzothiazol von der wäßrigen Phase abgetrennt.

EP 0 098 957 A2

0098957

## Verfahren zur Herstellung von kristallinem 2,6-Dichlorbenzothiazol

Die Erfindung betrifft ein Verfahren zur Herstellung von kristallinem 2,6-Dichlorbenzothiazol.

2,6-Dichlorbenzothiazol ist ein wichtiges Zwischenprodukt, insbesondere zur Herstellung von Pflanzenschutzmitteln und Arzneimitteln. Es läßt sich herstellen durch Chlorierung von 6-Chlor-2-merkaptobenzothiazol mit Chlorierungsmitteln, wie Chlor (DE-OS 16 70 453), Schwefelchloriden (US-PS 20 43 948), Sulfurylchlorid (Modica, Barni und Magrassi, Ann. Chimica 53 (1963), Nr. 6, Seiten 733 bis 740) und Phosgen (DE-AS 11 64 413). Weitere Herstellungsmethoden sind die Umwandlung von 6-Chlor-2-aminobenzothiazol in 2,6-Dichlorbenzothiazol (vgl. Modica, Barni und Magrassi loc.cit.) und der Umsatz von 2,4-Dichlorphenylisocyaniddichlorid mit elementarem Schwefel (DE-OS 30 18 088).

Bei der Weiterverarbeitung des 2,6-Dichlorbenzothiazols zu Pflanzenschutz- und Arzneimitteln ist eine hohe Reinheit des Produkts, in der Regel deutlich über 99 % Reingehalt, erforderlich. Bei allen bekannten Verfahren zur Herstellung von 2,6-Dichlorbenzothiazol fällt das Produkt jedoch zunächst zusammen mit Neben- oder Abfallprodukten an, von denen es zur Reinigung, zweckmäßigerweise durch Destillation, abgetrennt werden muß. Wegen des geforderten hohen Reinheitsgrads ist hierbei jedoch ein erheblicher destillativer Aufwand notwendig, was die Verfahren verteuert. Da das Produkt bei der Destillation als Schmelze anfällt, die bereits bei ca. 98°C erstarrt, ergeben sich für die Lagerung, den Versand und die Handhabung des Produkts weitere Probleme, wenn nicht die Schmelze direkt weiter verarbeitet werden kann, was jedoch häufig nicht der Fall ist. Die Handhabung und der Transport des 2,6-Dichlorbenzothiazols in geschmolzener Form erfordert heizbare Behälter und Apparate und einen zusätzlichen Energieaufwand. Besonders erschwerend kommt hinzu, daß geschmolzenes 2,6-Dichlorbenzothiazol gegenüber allen üblichen metallischen Werkstoffen, wie VA-Stahl, Hastelloy und Tantal korrodierende Eigenschaften besitzt. Daher müssen beheizbare Transportbehälter aus speziellen Werkstoffen, wie z.B. Emaille, beschaffen sein, was zusätzliche Kosten verursacht. Auch können handelsübliche Zerkleinerungsapparate für Schmelzen, wie z.B. Schabwalzen aus metallischen Werkstoffen, wegen den korrodierenden Eigenschaften des geschmolzenen 2,6-Dichlorbenzothiazols nicht verwendet werden.

Die Handhabung und der Transport von 2,6-Dichlorbenzothiazol in gelöster Form ist unwirtschaftlich, da das 2,6-Dichlorbenzothiazol in allen üblichen organischen Lösungsmitteln nur wenig löslich ist. Außerdem zeigen auch die Lösungen gegenüber Metallen korrodierende Eigenschaften und migrieren ferner durch Kunststoffbehälter aus üblichen kostengünstigen    Materialien, wie Poly-vinylchlorid, Polyethylen und Polypropylen.

Durch die vorliegende Erfindung ist es möglich, 2,6-Dichlorbenzothiazol in eine leicht handhabbare, lager- und transportfähige Form zu überführen. Bei der Durchführung des erfindungsgemäßen Verfahrens tritt außerdem ein erheblicher Reinigungseffekt auf, so daß die Anforderungen an die destillative Reinigung wesentlich vermindert werden können.

Das erfindungsgemäße Verfahren zur Herstellung von kristallisiertem 2,6-Dichlorbenzothiazol ist dadurch gekennzeichnet, daß geschmolzenes 2,6-Dichlorbenzothiazol mit Wasser vereinigt wird und danach auskristallisiertes 2,6-Dichlorbenzothiazol von der wäßrigen Phase abgetrennt wird. Die Vereinigung der beiden Komponenten erfolgt zweckmäßigerweise unter Durchmischung und kann auf verschiedene Weise durchgeführt werden. Normalerweise wird man eine Komponente vorlegen und die andere Komponente hinzufügen. Falls man das geschmolzene 2,6-Dichlorbenzothiazol vorlegt, wird man seine Temperatur nur knapp oberhalb des Schmelzpunktes einstellen und heißes Wasser von z.B. 90 bis 100°C hinzufügen. Gegebenenfalls kann bei der Vereinigung der Komponenten auch unter Überdruck gearbeitet werden. Dadurch kann Wasser mit einer Temperatur von mehr als 100°C verwendet werden. Die vereinigten Komponenten werden dann unter möglichst kräftiger Durchmischung abgekühlt, wodurch das 2,6-Dichlorbenzothiazol auskristallisiert und danach auf an sich bekannte Weise von der wäßrigen Phase abgetrennt wird.

Es ist jedoch vorteilhaft, Wasser vorzulegen und geschmolzenes 2,6-Dichlorbenzothiazol in das vorgelegte Wasser, zweckmäßigerweise unter Durchmischung, einzutragen. Die Temperatur des geschmolzenen 2,6-Dichlorbenzothiazols kann dabei zwischen dem Schmelzpunkt und dem Siedepunkt des 2,6-Dichlorbenzothiazols, d.h. also zwischen ca. 98°C und ca. 290°C liegen. Aus Energieersparnisgründen wird man jedoch zu hohe Temperaturen des geschmolzenen 2,6-Dichlorbenzothiazols zu vermeiden suchen und das geschmolzene 2,6-Dichlorbenzothiazol bei möglichst niederen Temperaturen in das Wasser eintragen. Eine Temperatur des geschmolzenen 2,6-Dichlorbenzothiazols nahe dem Schmelzpunkt erfordert zumeist die Isolierung von Leitungen und Apparateteilen gegen Wärmeverluste, um ein Auskristallisieren der Schmelze in den Leitungen und Apparaten zu verhindern. Ohne eine derartige Wärmeisolierung kann man zumeist auskommen, wenn man kurze Rohrleitungen verwendet und die Temperatur der Schmelze 110 bis 160°C, vorzugsweise 120 bis 145°C, beträgt. Vorzugsweise wird das geschmolzene 2,6-Dichlorbenzothiazol in fein verteilter Form in das Wasser eingetragen. Dies kann z.B. dadurch geschehen, daß die Schmelze über eine Düse eingetragen wird. Statt einer Düse kann selbstverständlich auch ein Sprühkopf, eine Sprühscheibe oder eine andere Vorrichtung für die Feinverteilung

einer Schmelze verwendet werden. Ein besonderer apparativer Aufwand ist dabei jedoch nicht erforderlich. Es ist z.B. völlig ausreichend, das geschmolzene 2,6-Dichlorbenzothiazol durch eine Düse, die sich z.B. 10 bis 100 cm, vorzugsweise 20 bis 50 cm, über der Wasseroberfläche befindet und eine Düsenöffnung von z.B. 0,5 bis 5 mm, vorzugsweise 1 bis 3,5 mm, besitzt, unter dem für den Transport der Schmelze benötigten Druck bzw. Unterdruck auszupressen oder auszusaugen. Über die Größe der Düsenöffnung läßt sich die Korngröße des anfallenden Kristallisats beeinflussen. Die Düsenöffnung kann auch unterhalb der Wasseroberfläche angeordnet sein.

Das vorgelegte Wasser wird während der Eintragung der Schmelze zweckmäßigerweise gerührt. Die Temperatur des vorgelegten Wassers kann von 0° bis 100°C variiert werden. Zweckmäßigerweise wird jedoch Wasser von Raumtemperatur vorgelegt, so daß das anfallende Kristallisat direkt nach dem Eintragen der Schmelze weiterverarbeitet werden kann, ohne daß vorher gekühlt werden muß. In heißem Wasser von z.B. 95 bis 100°C bildet sich zunächst ein 2-Phasensystem aus zwei flüssigen Phasen, das anschließend unter starker Durchmischung abgekühlt werden muß, um die Kristallisation zu erzielen.

Die Menge des Wassers kann in weiten Grenzen variiert werden. Eine zu gering bemessene Wassermenge erschwert jedoch die Rührbarkeit der anfallenden Suspension und macht zudem zusätzliche Maßnahmen zur Wärmeabfuhr notwendig. In der Regel liegt die untere Grenze bei 0,5 kg Wasser pro kg geschmolzenes 2,6-Dichlorbenzothiazol. Nach oben kann die Wassermenge beliebig vergrößert werden, z.B. bis zu 100 kg Wasser pro kg geschmolzenem 2,6-Dichlorbenzothiazol, so daß das Gewichtsverhältnis geschmolzenes 2,6-Dichlorbenzothiazol zu Wasser in der Regel 1 : (0,5 bis 100) beträgt. Noch mehr Wasser anzuwenden, ist zwar möglich, bringt aber statt Vorteile nur Nachteile wegen des großen Wasservolumens. Das bevorzugte Gewichtsverhältnis geschmolzenes 2,6-Dichlorbenzothiazol : Wasser beträgt 1 : (1 bis 3).

Nach der Beendigung der Eintragung des geschmolzenen 2,6-Dichlorbenzothiazols in das Wasser ist die Kristallisation in der Regel bereits beendet, und die Phasen können, falls Wasser von Raumtemperatur vorgelegt worden war, ohne vorherige Kühlung in an sich bekannter Weise voneinander getrennt werden. Die Abtrennung des Kristallisats von der wäßrigen Phase kann z.B. durch Filtrieren, Absaugen, Zentrifugieren oder Dekantieren erfolgen.

Die bei dem erfindungsgemäßen Verfahren anfallenden wasserfeuchten Kristalle sind hervorragend handhabbar und rieselfähig. Gewünschtenfalls können sie noch getrocknet werden, was z.B. durch Vakuumtrocknung oder durch azeotrope Entwässerung erfolgen kann. Für eine azeotrope Entwässerung wird man zweckmäßigerweise das organische Lösungsmittel verwenden, das für die Weiterverarbeitung des 2,6-Dichlorbenzothiazols vorgesehen ist. Dabei wird man normalerweise auch darauf achten, daß die dabei auftretenden Temperaturen unter dem Schmelzpunkt des Produkts liegen. Es ist jedoch auch möglich, ein Lösungsmittel zu verwenden, welches das 2,6-Dichlorbenzothiazol löst oder die Entwässerung bei Temperaturen oberhalb des Schmelzpunktes des 2,6-Dichlorbenzothiazols mit einem entsprechend hoch siedenden Lösungsmittel vorzunehmen. In diesen Fällen wird eine wasserfreie Lösung oder Schmelze des gereinigten Produkts erhalten, was vorteilhaft sein kann, falls das Produkt in dieser Form direkt weiterverarbeitet wird.

Mit dem erfindungsgemäßen Verfahren ist ein Reinigungseffekt verbunden, der weiter verstärkt wird, wenn das Wasser einen basisch reagierenden Stoff und/oder ein Tensid enthält, das die Oberflächenspannung des Wassers herabsetzt. In der Regel sind Konzentrationen bis zu 10 Gew.% von dem basisch reagierenden Stoff und/oder bis zu 3 Gew.% von dem Tensid ausreichend. Geeignete basisch reagierende Stoffe sind z.B. Alkalihydroxide, wie Natrium- oder Kaliumhydroxid, Erdalkalihydroxid, wie Barium- oder Calciumhydroxid, Alkalicarbonate, wie z.B. Natrium- oder Kaliumcarbonat, Ammoniak und organische Amine. Ammoniak und Alkalihydroxide werden bevorzugt. Häufig genügen bereits Konzentrationen bis 2 Gew.% an dem basisch reagierenden Stoff. Geeignete, die Oberflächenspannung des Wassers herabsetzende Tenside sind z.B. handelsübliche Netzmittel, wie Seifen, Seifenrindenextrakte, Türkischrotöle, Fettalkoholsulfate, Alkylarylsulfonate, Invertseifen, ethoxylierte Acetylenalkohole und -diole etc. Normalerweise ist von diesen Netzmitteln eine Konzentration bis 1 Gew.% ausreichend. Selbstverständlich können auch Mischungen von 2 oder mehreren basisch reagierenden Stoffen und/oder 2 oder mehreren Tensiden eingesetzt werden.

Nach dem erfindungsgemäßen Verfahren wird, z.B. ausgehend von einem ca. 95 %igen 2,6-Dichlorbenzothiazol, ein kristallisiertes 2,6-Dichlorbenzothiazol mit einer gaschromatographisch bestimmten Reinheit von über 99 % erhalten. Bei Verwendung von Wasser, das einen basisch reagierenden Stoff und/oder ein die Oberflächenspannung des Wassers herabsetzendes Tensid enthält, werden gaschromatographisch bestimmte Reinheiten von über 99,5 % erzielt. Die nach der Abtrennung des kristallisierten 2,6-Dichlorbenzothiazols zurückbleibende wäßrige Phase kann ohne weiteres für weitere Ansätze ohne Qualitätseinbuße mehrfach verwendet werden.

Die bei dem erfindungsgemäßen Verfahren anfallenden wasserfeuchten 2,6-Dichlorbenzothiazol-Kristalle besitzen eine Haltbarkeit von mindestens 3 Monaten ohne Gehaltsabfall, sind rieselfähig, nicht staubend und deshalb hervorragend handhabbar. Bei einer 3 Monate langen Lagerung der wasserfeuchten Kristalle in Behältern aus Weißblech trat keine Korrosion auf, was als außerordentlich überraschend bezeichnet werden muß. Die wasserfeuchten 2,6-Dichlorbenzothiazol-Kristalle können auch 4 Wochen bei 50°C in Polypropylen-Behältern gelagert werden, ohne daß eine Migration durch die Behälterwand auftritt.

In den nachfolgenden Beispielen bedeuten, sofern nichts anderes angegeben ist, Prozente Gewichtsprozente.

Beispiel 1

In einem 100-l-Rührwerksbehälter aus Emaille werden 50 l Wasser mit einem Gehalt von 1 % Natriumhydroxid bei Raumtemperatur vorgelegt und gerührt (Rührerdrehzahl: 120 Umdrehungen/min). 40 cm oberhalb des Wasserspiegels ist eine Düse mit einer Düsenöffnung von 2mm angeordnet, durch die 30 kg geschmolzenes 2,6-Dichlorbenzothiazol (hergestellt nach dem Verfahren der DE-OS 3 018 088) mit einer Temperatur von 140°C und einem Reinheitsgehalt von 96,1 % eingesaugt werden. Dies geschieht durch Anlegen eines Vakuums von ca. 530 mbar, wodurch die Schmelze aus einem Vorratsbehälter über eine kurze, nicht beheizte Leitung innerhalb von 10 min in den Rührwerksbehälter eingesaugt wird. Danach ist die Temperatur der Wasserphase in dem nicht gekühlten Rührwerksbehälter auf 40°C angestiegen. Die entstandene Kristallsuspension wird sofort über ein Filter abgelassen und mit 10 l Wasser nachgewaschen. Man erhält 2,6-Dichlorbenzothiazol in rieselfähiger kristalliner Form, das nach der Trocknung eine gaschromatographisch bestimmte Reinheit von 99,9 % aufweist;Trockenausbeute 28,7 kg.

Bei einer zweimaligen Wiederholung des Ansatzes unter jeweiliger Wiederverwendung der abgetrennten wäßrigen Phase werden keine Qualitätseinbußen festgestellt.

### Beispiel 2

Beispiel 1 wird wiederholt, wobei jedoch 50 l Trinkwasser vorgelegt werden und auf ein Nachwaschen der abgetrennten Kristalle verzichtet wird. Das erhaltene kristallisierte Produkt besitzt nach dem Trocknen eine gaschromatographische bestimmte Reinheit von über 99,2 %.

### Beispiel 3

Beispiel 1 wird wiederholt, wobei 50 l einer 2%igen wäßrigen Ammoniaklösung vorgelegt werden. Das erhaltene kristallisierte Produkt besitzt nach dem Trocknen eine gaschromatographisch bestimmte Reinheit von über 99,6 %.

### Beispiel 4

Beispiel 1 wird wiederholt, wobei 50 l Trinkwasser vorgelegt werden, die 1 % eines handelsüblichen Netzmittels auf der Basis eines sulfonierten Diisobutylamids der Ölsäure enthält. Das erhaltene kristallisierte Produkt besitzt nach dem Trocknen eine gaschromatographisch bestimmte Reinheit von über 99,5 %.

### Beispiel 5

Beispiel 1 wird wiederholt, wobei eine Schmelze von 97-%igem 2,6-Dichlorbenzothiazol (hergestellt nach dem Verfahren der DE-AS 16 70 453) zum Einsatz kommt. Das erhaltene kristallisierte Produkt besitzt nach dem Trocknen eine gaschromatographisch bestimmte Reinheit von über 99,6 %.

## PATENTANSPRÜCHE

1. Verfahren zur Herstellung von kristallisiertem 2,6-Dichlorbenzothiazol, dadurch gekennzeichnet, daß geschmolzenes 2,6-Dichlorbenzothiazol mit Wasser vereinigt und danach auskristallisiertes 2,6-Dichlorbenzothiazol von der wäßrigen Phase abgetrennt wird.

2. Verfahren nach Anspruch 1, dadurch gekennzeichnet, daß die Vereinigung unter Durchmischung erfolgt.

3. Verfahren nach Anspruch 1 und/oder 2, dadurch gekennzeichnet, daß geschmolzenes 2,6-Dichlorbenzothiazol in Wasser eingetragen wird.

4. Verfahren nach Anspruch 3, dadurch gekennzeichnet, daß geschmolzenes 2,6-Dichlorbenzothiazol eingetragen wird, das eine Temperatur von 110 bis 160°C besitzt.

5. Verfahren nach Anspruch 3 und/oder 4, dadurch gekennzeichnet, daß geschmolzenes 2,6-Dichlorbenzothiazol eingetragen wird, das eine Temperatur von 120 bis 145°C besitzt.

6. Verfahren nach einem oder mehreren der Ansprüche 1 bis 5, dadurch gekennzeichnet, daß ein Gewichtsverhältnis geschmolzenes 2,6-Dichlorbenzothiazol : Wasser = 1 : (0,5 bis 100) eingehalten wird.

7. Verfahren nach einem oder mehreren der Ansprüche 1 bis 6, dadurch gekennzeichnet, daß ein Gewichtsverhältnis geschmolzenes 2,6-Dichlorbenzothiazol : Wasser = 1 : (1 : 3) eingehalten wird.

8. Verfahren nach einem oder mehreren der Ansprüche 3 bis 7, dadurch gekennzeichnet, daß das geschmolzene 2,6-Dichlorbenzothiazol in fein verteilter Form in Wasser eingetragen wird.

9. Verfahren nach einem oder mehreren der Ansprüche 1 bis 8, dadurch gekennzeichnet, daß das Wasser bis zu 10 Gew.% eines basisch reagierenden Stoffs enthält.

10. Verfahren nach einem oder mehreren der Ansprüche 1 bis 9, dadurch gekennzeichnet, daß das Wasser bis zu 3 Gew.% eines Tensids enthält, das die Oberflächenspannung des Wassers herabsetzt.